# EUROPEAN PATENT APPLICATION

(11) **EP 3 936 089 A1**
(43) Date of publication of application: **12.01.2022**
(21) Application number: 20766590.2
(22) Date of filing: 30.01.2020
(51) Int. Cl.: A61F 2/966

(54) **STENT DELIVERY SYSTEM**

(30) Priority: 05.03.2019 JP 2019039575
(71) Applicant: ASAHI INTECC CO., LTD., Seto-shi, Aichi 489-0071 (JP)
(72) Inventor: FUSEYA, Yukihiro, Seto-shi, Aichi 489-0071 (JP); TSUZUKU, Marina, Seto-shi, Aichi 489-0071 (JP)
(74) Representative: Winter, Brandl - Partnerschaft mbB
(86) International application number: PCT/JP2020/003348
(87) International publication number: WO 2020/179293

(57) **Abstract**

A stent delivery system 1 includes a distal end shaft 11 has a tapered portion 11b, a spirally-arranged protruding portion 21 provided on an outer peripheral face of the tapered portion 11b, an inner shaft 31 extending toward a proximal end side of the distal end shaft 11 in a long axis direction, a self-expanding stent 41, and an outer shaft 51, and the outer shaft 51 is slidable between a first position where the stent 41 in a contracted state is covered and a second position on the proximal end side of the stent 41 in the long axis direction of the inner shaft 31, and an outermost periphery of the outer shaft 51 fits inside an outermost periphery of the spirally-arranged protruding portion 21 in a frontal view from the distal end side of the distal end shaft 11 in the long axis direction.

## Description

### TECHNICAL FIELD

The disclosed embodiments relate to a stent delivery system.

### BACKGROUND ART

As a device for widening a penetration-pore or a constricted part of a blood vessel, a digestive tract, or the like caused by a lesion or the like, for example, a stent obtained by weaving a metal wire in a mesh shape is known.

When widening a constricted part caused in a blood vessel, a digestive tract, or the like using such a stent, it is necessary to preliminarily widen the constricted part to a size allowing insertion of the stent in a diameter-decreased state prior to placement of the stent in the constricted part. As an instrument used for such preliminary manipulation, for example, dilators have been proposed.

A dilator has a tapered distal end portion that increases in diameter toward a proximal end side, and when the tapered part passes through a constricted part or the like, this constricted part or the like is widened (e.g. see Patent Literature 1). Then, the stent in a diameter-decreased state is inserted into the widened constricted part or the like, then this stent is widened outward in a radial direction and placed in the constricted part, so that the constricted part of the blood vessel, the digestive tract, or the like can be stably widened.

### CITATION LIST

### Patent Literature

Patent Literature 1: Japanese Patent Laid-Open No. 2008-11867

### SUMMARY OF INVENTION

### Problem to be Solved

However, when using the conventional dilator as described above, it is necessary to transport the stent to the constricted part or the like after drawing the dilator out of the preliminarily widened constricted part or the like. Thus, the preliminarily widened constricted part or the like may be constricted again in a body cavity such as a significantly elastic blood vessel or digestive tract, and smooth operation may be disturbed by necessity of additional preliminary operation, or the like.

The disclosed embodiments were made based on the above circumstances, and an object of the disclosed embodiments is to provide a stent delivery system capable of smoothly placing a stent in a body cavity such as a blood vessel and a digestive tract.

### Solution to Problem

This disclosure includes some aspects:
(1) a stent delivery system including a distal end shaft that has an inner cavity, and a tapered portion having an outer diameter gradually increasing from a distal end to a proximal end, a spirally-arranged protruding portion that is provided on an outer peripheral face of the tapered portion and has a gap between adjacent sections along a long axis direction of the distal end shaft (hereinafter, also simply referred to as "long axis direction"), an inner shaft that is connected to the proximal end of the distal end shaft, has an inner cavity communicating with the inner cavity of the distal end shaft, and extends toward the proximal end side in the long axis direction of the distal end shaft, a self-expanding stent that covers the inner shaft and is expandable and contractable in a radial direction of the inner shaft, and an outer shaft that covers the inner shaft and is slidable along a long axis direction of the inner shaft, wherein the outer shaft is slidable between a first position where the stent in a contracted state is covered and a second position on the proximal end side of the stent in the long axis direction of the inner shaft, and an outermost periphery of the outer shaft fits inside an outermost periphery of the spirally-arranged protruding portion in a frontal view from the distal end side of the distal end shaft in the long axis direction; and
(2) the stent delivery system according to (1), wherein an outer diameter of the proximal end of the distal end shaft and an outer diameter of the distal end of the outer shaft substantially coincide with each other, and the proximal end of the distal end shaft and the distal end of the outer shaft abuts on each other such that an outer peripheral edge of the proximal end of the distal end shaft and an outer peripheral edge of the distal end of the outer shaft coincide with each other at the first position.

In the present specification, the "distal end side" means a direction along the long axis direction of the distal end shaft, in which the distal end shaft is located with respect to the inner shaft. In addition, the "proximal end side" means a direction along the long axis direction of the distal end shaft, which is a direction opposite to the distal end side. In addition, the "distal end" refers to a distal end portion of any member or part, and the "proximal end" refers to a proximal end portion of any member or part. The "outermost periphery" means a shape formed by an outline of a particular member (spirally-arranged protruding portion, outer shaft, etc.) as viewed from the long axis direction of the distal end shaft.

### Effects of the Invention

The disclosed embodiments can provide a stent delivery system capable of smoothly placing a stent in a body cavity such as a blood vessel.

### BRIEF DESCRIPTION OF DRAWINGS

FIG. 1 is a schematic side view of an embodiment of the disclosed embodiments, illustrating a state that an outer shaft is at a first position.
FIG. 2 is a schematic front view of FIG. 1.
FIG. 3 is a schematic side view of an embodiment of the disclosed embodiments, illustrating a state that an outer shaft is at a second position.
FIG. 4A is a schematic side view illustrating an example of a usage manner in the embodiment of FIG. 1.
FIG. 4B is a schematic side view illustrating an example of a usage manner in the embodiment of FIG. 1.
FIG. 4C is a schematic side view illustrating an example of a usage manner in the embodiment of FIG. 1.
FIG. 4D is a schematic side view illustrating an example of a usage manner in the embodiment of FIG. 1.
FIG. 4E is a schematic side view illustrating an example of a usage manner in the embodiment of FIG. 1.
FIG. 4F is a schematic side view illustrating an example of a usage manner in the embodiment of FIG. 1.
FIG. 5A is a schematic side view of a modification example of FIG. 1, illustrating a state that the outer shaft is at the first position.
FIG. 5B is a schematic side view of a modification example of FIG. 1, illustrating a state that the outer shaft is at the first position.
FIG. 5C is a schematic side view of a modification example of FIG. 1, illustrating a state that the outer shaft is at the first position.

### EMBODIMENTS OF THE INVENTION

The stent delivery system includes: a distal end shaft that has an inner cavity and a tapered portion having an outer diameter gradually increasing from a distal end to a proximal end; a spirally-arranged protruding portion that is provided on an outer peripheral face of the tapered portion and has a gap between adjacent sections along a long axis direction of the distal end shaft; an inner shaft that is connected to the proximal end of the distal end shaft, has an inner cavity communicating with the inner cavity of the distal end shaft, and extends toward the proximal end side of the distal end shaft in the long axis direction; a self-expanding stent that covers the inner shaft and is expandable and contractable in a radial direction of the inner shaft; and an outer shaft that covers the inner shaft and is slidable along a long axis direction of the inner shaft: and is characterized in that the outer shaft is slidable between a first position where the stent in a contracted state is covered and a second position on the proximal end side of the stent in the long axis direction of the inner shaft, and an outermost periphery of the outer shaft fits inside an outermost periphery of the spirally-arranged protruding portion in a frontal view from the distal end side of the distal end shaft in the long axis direction.

Hereinafter, an embodiment of the disclosed embodiments will be explained with reference to the figures, but the disclosed embodiments are not limited only to the embodiments illustrated in the figures. Note that the dimensions of the stent delivery system indicated in the figures are illustrated for the purpose of facilitating understanding of the implementation details, and do not correspond to the actual dimensions.

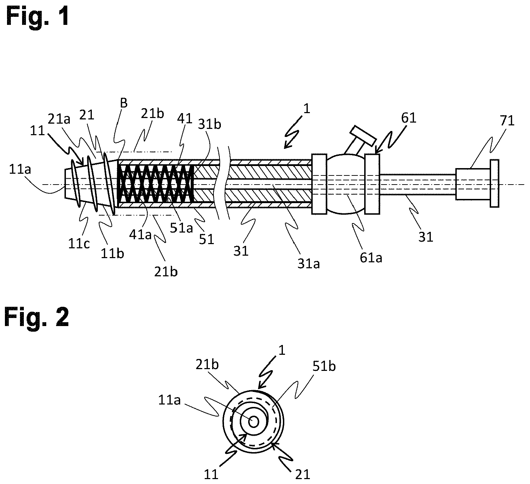

FIG. 1 is a schematic side view of an embodiment of the disclosed embodiments, illustrating a state that the outer shaft is at the first position. FIG. 2 is a schematic front view of FIG. 1. As illustrated in FIG. 1 and FIG. 2, a stent delivery system 1 is schematically composed of a distal end shaft 11, a spirally-arranged protruding portion 21, an inner shaft 31, a stent 41, an outer shaft 51, and a connector 61.

The distal end shaft 11 has an inner cavity 11a, and a tapered portion 11b having an outer diameter gradually increasing from a distal end to a proximal end. Specifically, the inner cavity 11a can be composed of e.g. a through-hole passing from the distal end to the proximal end. As the tapered portion 11b, it is possible to adopt a tapered portion that has e.g. an outer periphery shape gradually increasing in diameter from the distal end to the proximal end (see FIG. 1), a tapered portion having a diameter gradually increasing from the distal end to the proximal end (not illustrated), and a combination thereof (not illustrated), or the like. For example, a guide wire not illustrated is inserted into the inner cavity 11a, and the stent delivery system 1 advances and retreats along the guide wire.

A material constituting the distal end shaft 11 preferably has antithrombogenicity, flexibility, and biocompatibility because the shaft is inserted into a body cavity. For example, it is possible to adopt a resin material such as a polyamide resin, a polyolefin resin, a polyester resin, a polyurethane resin, a silicone resin, and a fluororesin; a metal material such as a stainless steel and a superelastic alloy (nickel-titanium alloy), and the like.

The distal end shaft 11 may have various coating films (not illustrated) on a side of an outer peripheral face 11c of the distal end shaft 11. Examples of such coating films include a protective film (typified by a plating film) for protecting the surface of the distal end shaft 11, a base film for improving adhesiveness between the distal end shaft 11 and a spirally-arranged protruding portion 21 described later, and the like.

The spirally-arranged protruding portion 21 is provided on the outer peripheral face 11c of the tapered portion 11b and has a gap 21a between adjacent sections along the long axis direction of the distal end shaft 11 (adjacent protruding portions 21 are separated in the long axis direction). Specifically, for example, this spirally-arranged protruding portion 21 protrudes from the outer peripheral face 11c of the distal end shaft 11 outward in a radial direction of this distal end shaft 11, and is formed such that the outermost periphery of the spirally-arranged protruding portion 21 (see the two-dot chain line 21b in FIG. 1) is located on the radial outside of the outermost periphery of the distal end shaft (in this embodiment, the outer shape of the proximal end of the distal end shaft 11) in a frontal view from the distal end side in the long axis direction of the stent delivery system 1.

The spirally-arranged protruding portion 21 can be formed as a continuous or intermittent single-thread or multi-thread protruding portion. In addition, the spirally-arranged protruding portion 21 can also be formed by spirally winding one or more wires around the outer peripheral face 11c of the distal end shaft 11. The spirally-arranged protruding portion 21 may be either integrated with or separated from the distal end shaft 11. In this embodiment, the spirally-arranged protruding portion 21 is formed as a continuous single-thread protruding portion so as to be integrated with the distal end shaft 11 by casting or the like.

A part of the distal end shaft 11, where the spirally-arranged protruding portion 21 is formed may be any part between the distal end and the proximal end of the distal end shaft 11 in the long axis direction. For example, it is possible to adopt a configuration in which the spirally-arranged protruding portion is formed entirely from the distal end to the proximal end of the distal end shaft 11 (see FIG. 1), a configuration in which the spirally-arranged protruding portion is formed from the distal end to a middle of the distal end shaft (not illustrated), a configuration in which the spirally-arranged protruding portion is formed from the middle to the proximal end of the distal end shaft (not illustrated), and the like.

Preferably, the spirally-arranged protruding portion 21 does not constitute a blade (shaped not to cut biological tissues). That means, the spirally-arranged protruding portion 21 preferably has a transverse section (section orthogonal to the spiral direction of the spirally-arranged protruding portion 21) in which an outside end portion (apex portion) of the distal end shaft 11 in the radial direction is not an acute corner portion. Examples of such an end portion include an end portion composed of an obtuse corner, an end portion composed of a corner portion shaped so as to include a curve (e.g. a curve including a part of a circle or an ellipse, etc.), and the like. Thereby, the stent delivery system 1 can widen a hole of an object without damaging biological tissues on an inner wall face of the hole of the object when performing a preliminary operation (preliminary widening of a constricted part or the like before inserting a diameter-decreased stent).

When the spirally-arranged protruding portion and the distal end shaft are separately formed, for example, the same material as the material constituting the aforementioned distal end shaft, or the like can be adopted as a material constituting the spirally-arranged protruding portion 21.

The inner shaft 31 is connected to the proximal end of the distal end shaft 11, has an inner cavity 31a communicating with the inner cavity 11a of the distal end shaft 11, and extends toward the proximal end side of the distal end shaft 11 in the long axis direction. Specifically, the inner shaft 31 is composed of e.g. a through-hole through which the inner cavity 31a passes from the distal end to the proximal end. The outer peripheral face of the inner shaft 31 has a hollow cylindrical face on at least a part in the long axis direction so as to hold the stent 41 (described later). The outer peripheral face of the inner shaft 31 may have a lock portion 31b for locking the proximal end of the stent 41. A proximal end-side part of the outer peripheral face of the inner shaft 31 can be formed in a shape suited to an inner peripheral face of an inner cavity 61a of the connector 61 (described later) so as to hold the connector 61 slidably. A handle 71 for rotating the distal end shaft 11 via the inner shaft 31 is connected to the proximal end of the inner shaft 31. The inner shaft 31 and the proximal end of the distal end shaft 11 can be connected to each other, and the proximal end of the inner shaft 31 and the handle 71 can be connected to each other, e.g. by jointing using welding, adhesion using an adhesive, or the like.

As a material constituting the inner shaft 31, for example, the same material as the material constituting the aforementioned distal end shaft 11, or the like can be adopted, because the shaft is inserted into the body cavity.

The stent 41 is a self-expanding member that covers the inner shaft 31 and is expandable and contractable in the radial direction of this inner shaft 31. Specifically, as this stent 41, for example, a stent woven into a mesh shape using one or a plurality of metal wires 41a (see FIG. 1), a stent formed into a mesh shape by cutting a part of a substantially-hollow cylindrical body and boring holes thereon using laser processing or the like (not illustrated), and the like can be adopted.

As a material constituting the stent 41, for example, the same material as the material constituting the aforementioned distal end shaft 11, or the like can be adopted, because the stent is placed in the body cavity.

The outer shaft 51 covers the inner shaft 31 and is slidable along the long axis direction of the inner shaft 31. Specifically, the outer shaft 51 can be composed of e.g. a hollow cylindrical member having a through-hole formed from the distal end to the proximal end such that the stent 41 is wrapped (supported) by an inner peripheral face 51a of the outer shaft 51 and the outer shaft 51 can slide along the long axis direction of the inner shaft 31.

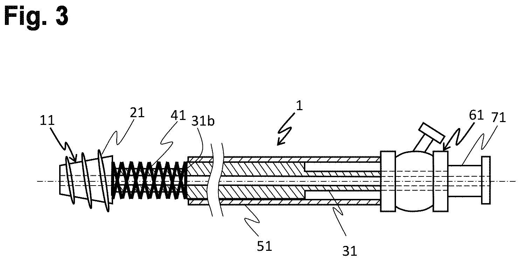

The outer shaft 51 slides in the long axis direction of the inner shaft 31 between the first position where the stent 41 in a contracted state is covered and the second position on the proximal end side of the stent 41. The first position refers to a position where the entire stent 41 in the contracted state (non-expanded state) is housed inside the outer shaft 51 (see FIG. 1), and the second position refers to a position where the outer shaft 51 is located on the proximal end side of the first position and the stent 41 is completely exposed to the outside (see FIG. 3). The outer shaft 51 can slide e.g. by connecting the proximal end of the outer shaft 51 to the connector 61 and moving the connector 61 forward and backward along the long axis direction.

An outermost periphery 51b (having the same distal outer shape as of the outer shaft 51, in this embodiment) of the outer shaft 51 is arranged to fit inside the outermost periphery (see the two-dot chain line 21b in FIG. 1) of the spirally-arranged protruding portion 21 in a frontal view from the distal end side in the long axis direction of the distal end shaft 11.

Herein, it is preferable that the outer diameter of the proximal end of the distal end shaft 11 and the outer diameter of the distal end of the outer shaft 51 substantially coincide with each other, and the proximal end of the distal end shaft 11 and the distal end of the outer shaft 51 abuts on each other such that an outer peripheral edge of the proximal end of the distal end shaft 11 and an outer peripheral edge of the distal end of the outer shaft 51 coincide with each other at the first position (see FIG. 1). Thereby, for example, when a boundary portion B between the distal end shaft 11 and the outer shaft 51 passes through a constricted part or the like, the outer shaft 51 can be advanced while the boundary portion B is not caught by an inner wall of the constricted part or the like, resulting in smooth placement of the stent 41.

As a material constituting the outer shaft 51, for example, the same material as the material constituting the aforementioned distal end shaft 11, or the like can be adopted, because the shaft is inserted into the body cavity.

The connector 61 is a part at which an operator operates the stent delivery system 1. This connector 61 has e.g. the through-hole 61a along the long axis direction and can be configured such that the inner shaft 31 penetrates this through-hole 61a. The outer shape of the connector 61 is not particularly limited as long as the effects of the disclosed embodiments are not impaired. The connector 61 can be connected to e.g. the proximal end of the outer shaft 51 by jointing using welding, adhesion using an adhesive, or the like.

As for the dimensions of each portion in the stent delivery system 1, the distal end shaft 11 ordinarily has outer diameters of 0.8 to 3.0 mm on the distal end and 1.4 to 5.0 mm on the proximal end. The spirally-arranged protruding portion 21 has a maximum diameter of 1.6 to 6.0 mm on the outermost periphery. The outer shaft 51 has an outer diameter of 1.4 to 6.0 mm. The stent 41 in a contracted state has an outer diameter of 0.7 to 2.0 mm and a length of 50 to 250 mm. The inner cavities 11a and 31a of the distal end shaft 11 and the inner shaft 31 have an inner diameter of 0.5 to 1.5 mm.

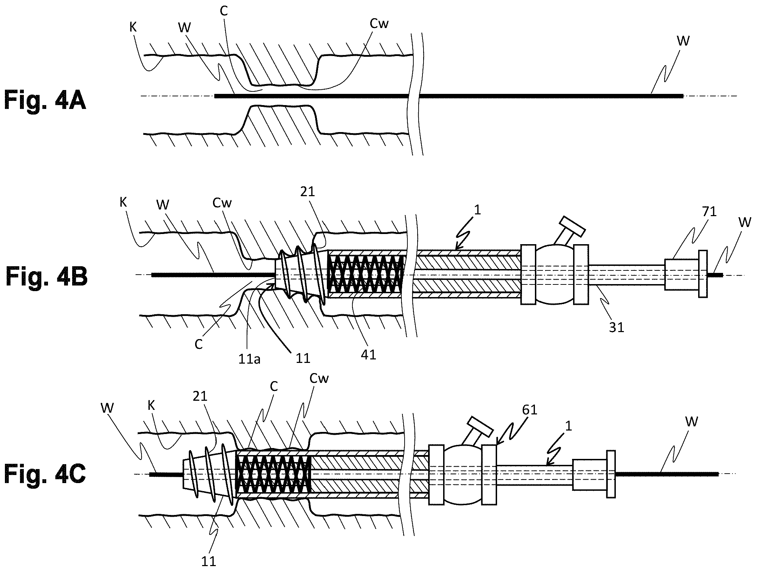

Next, an example of how to use the stent delivery system 1 will be explained with reference to FIG. 4A to FIG. 4F. Herein, an example of a procedure in which a constricted part C caused in a digestive tract K such as a bile duct and a pancreatic duct due to a lesion is preliminarily widened, and then the stent 41 is placed in the constricted part C will be explained.

First, a guide wire W is inserted into the constricted part C prior to insertion of the stent delivery system 1. (see FIG. 4A).

Next, the proximal end of the guide wire W is inserted into the inner cavity 11a of the stent delivery system 1 so as to protrude from the proximal end of the stent delivery system 1, and then this stent delivery system 1 is pushed forward to the immediate vicinity of the constricted part C along the guide wire W. Subsequently, the distal end shaft 11 is inserted into the constricted part C from the distal end, then handle 71 is rotated to advance the stent delivery system 1 while the spirally-arranged protruding portion 21 is screwed into an inner wall Cw of the constricted part C (see FIG. 4B), and the advancement of the stent delivery system 1 is stopped at a position where the stent 41 enters the constricted part C (see FIG. 4C). Thereby, the preliminary widening of the constricted part C is completed.

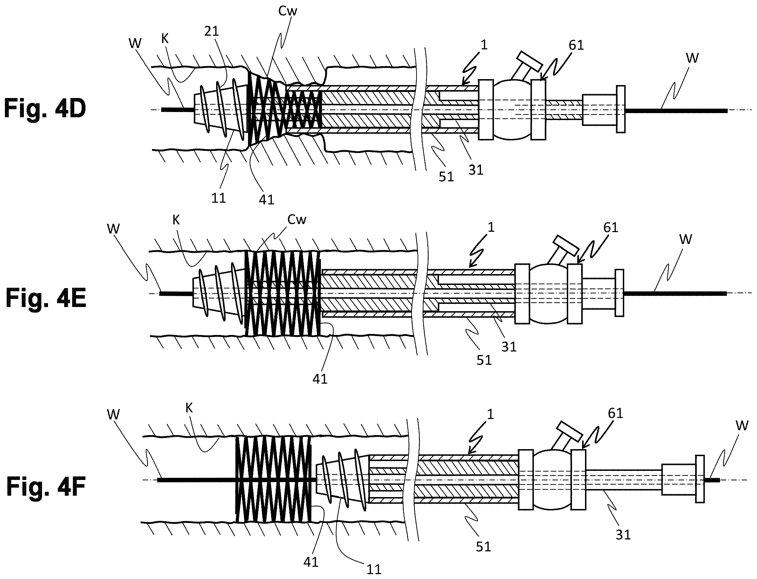

Subsequently, the stent 41 possessed by the stent delivery system 1 is separated from the stent delivery system 1 to widen the preliminarily widened constricted part C. Specifically, the outer shaft 51 is moved (slid) from the first position to the second position by pulling the connector 61 toward the proximal end side with respect to the inner shaft 31. The stent 41 is gradually exposed as the outer shaft 51 is moved from the first position to the second position. The gradually exposed part of the stent 41 expands outward in the radial direction owing to the self-expanding action of the stent 41 (see FIG. 4D). In a state that the stent 41 is fully exposed to the outside in the second position, the stent 41 increases in diameter as a whole to widen the inner wall Cw of the constricted part C outward in the radial direction (see FIG. 4E). Subsequently, the distal end shaft 11 is drawn out toward the proximal end side so as to pass through the inside of the self-expanded stent 41 (see FIG. 4F), and the stent delivery system 1 from which the stent 41 is separated is removed out of the body. Thereby, the series of procedures for placing the stent 41 in the digestive tract K is completed.

As described above, since the stent delivery system 1 has the aforementioned configuration, widening of the constricted part C and placement of the stent 41 can be continuously performed, and the stent 41 can be smoothly placed in a body cavity such as the digestive tract K.

Note that the disclosed embodiments are not limited to the configurations of the aforementioned embodiments, but is stipulated by claims, and the disclosed embodiments are intended to include all modifications within the meaning and scope equivalent to those in claims.

For example, in the aforementioned embodiments, the stent delivery system 1 in which the outer peripheral edge of the proximal end of the distal end shaft 11 and the outer peripheral edge of the distal end of the outer shaft 51 coincide with each other at the first position, has been explained, but the stent delivery system 1 may have any aspect as long as the outermost periphery of the outer shaft 51 fits inside the outermost periphery of the spirally-arranged protruding portion 21 in a frontal view.

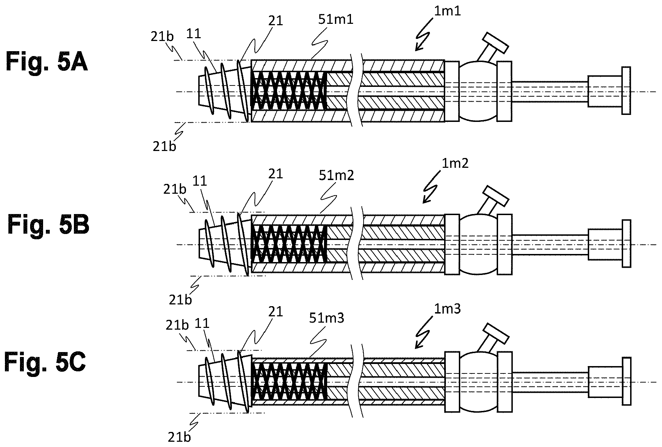

Examples of such a stent delivery system include a stent delivery system 1m1 in which an outermost periphery of an outer shaft 51m1 has the same shape as of the outermost periphery of the spirally-arranged protruding portion 21 in a frontal view (see FIG. 5A), a stent delivery system 1m2 in which an outermost periphery of an outer shaft 51m2 is located inside the outermost periphery of the spirally-arranged protruding portion 21 and outside the outer peripheral edge of the proximal end of the distal end shaft 11 (see FIG. 5B), a stent delivery system 1m3 in which an outermost periphery of an outer shaft 51m3 is located inside the outermost periphery of the spirally-arranged protruding portion 21 and inside the outer peripheral edge of the proximal end of the distal end shaft 11 (see FIG. 5C), and the like.

### DESCRIPTION OF REFERENCE NUMERALS

- 1: Stent delivery system
- 11: Distal end shaft
- 11b: Inner cavity
- 11b: Tapered portion
- 21: Spirally-arranged protruding portion
- 31: Inner shaft
- 31a: Inner cavity
- 41: Stent
- 51: Outer shaft

## Claims

1. A stent delivery system comprising
a distal end shaft that has an inner cavity, and a tapered portion having an outer diameter gradually increasing from a distal end to a proximal end,
a spirally-arranged protruding portion that is provided on an outer peripheral face of the tapered portion and has a gap between adjacent sections along a long axis direction of the distal end shaft,
an inner shaft that is connected to the proximal end of the distal end shaft, has an inner cavity communicating with the inner cavity of the distal end shaft, and extends toward the proximal end side in the long axis direction of the distal end shaft,
a self-expanding stent that covers the inner shaft and is expandable and contractable in a radial direction of the inner shaft, and
an outer shaft that covers the inner shaft and is slidable along a long axis direction of the inner shaft, wherein
the outer shaft is slidable between a first position where the stent in a contracted state is covered and a second position on the proximal end side of the stent in the long axis direction of the inner shaft, and
an outermost periphery of the outer shaft fits inside an outermost periphery of the spirally-arranged protruding portion in a frontal view from the distal end side of the distal end shaft in the long axis direction.

2. The stent delivery system according to claim 1, wherein
an outer diameter of the proximal end of the distal end shaft and an outer diameter of the distal end of the outer shaft substantially coincide with each other, and
the proximal end of the distal end shaft and the distal end of the outer shaft abuts on each other such that an outer peripheral edge of the proximal end of the distal end shaft and an outer peripheral edge of the distal end of the outer shaft coincide with each other at the first position.
